# EUROPEAN PATENT SPECIFICATION

(11) **EP 1 830 856 B1**
(45) Date of publication and mention of the grant of the patent: **18.04.2012**
(21) Application number: 05782582.0
(22) Date of filing: 02.08.2005
(51) Int. Cl.: A61K 31/02, A61P 25/00, A61P 23/00, A61K 45/06, A61K 33/00, A61P 39/00, A61P 41/00

(54) **PREVENTING PATHOLOGICAL INCREASES IN THE RATE OF NERVE CELL SUICIDE IN IMMATURE NERVOUS SYSTEMS**
VERHINDERUNG VON PATHOLOGISCHEN ANSTIEGEN DER NERVENZELLSUIZIDRATE IN UNREIFEN NERVENSYSTEMEN
PREVENTION D'AUGMENTATIONS PATHOLOGIQUES DU SUICIDE DE LA CELLULE NERVEUSE DANS DES SYSTEMES NERVEUX IMMATURES

(30) Priority: 02.08.2004 US 598390 P
(43) Date of publication of application: 12.09.2007
(73) Proprietor: Olney, John W., Ladue MO 63124 (US)
(72) Inventor: Olney, John W., Ladue MO 63124 (US)
(74) Representative: Hards & Franke
(86) International application number: PCT/US2005/027460
(87) International publication number: WO 2006/017524

(56) References cited:
- WO-A-2006/018655
- US-A1- 2003 180 375
- US-A1- 2004 028 656
- US-B1- 6 559 190
- US-B1- 6 559 190
- BEALS J K ET AL: "Melatonin reduces apoptosis resulting from combined nitrous oxide, midazolam, and isoflurane anesthesia in 7 - day old rat pups" ABSTRACTS OF THE SOCIETY FOR NEUROSCIENCE, SOCIETY FOR NEUROSCIENCE, WASHINGTON, DC, US, vol. 2003, 8 November 2003 (2003-11-08), page ABSTRACTNO6356, XP008088682 ISSN: 0190-5295
- DIAMOND B I ET AL: "Potential of lithium as anesthetic premedicant" LANCET 1977 UNITED KINGDOM, vol. 2, no. 8050, 1977, pages 1229-1230, XP002469782 ISSN: 0140-6736
- SCALFARO P ET AL: "Salbutamol prevents the increase of respiratory resistance caused by tracheal intubation during sevoflurane anesthesia in asthmatic children" ANESTHESIA AND ANALGESIA, WILLIAMS AND WILKINS, BALTIMORE, MD, US, vol. 93, no. 4, October 2001 (2001-10), pages 898-902, XP008088605 ISSN: 0003-2999
- POHL D ET AL: "N-methyl-D-aspartate antagonists and apoptotic cell death triggered by head trauma in developing rat brain" PROCEEDINGS OF THE NATIONAL ACADEMY OF SCIENCES OF THE UNITED STATES OF AMERICA 02 MAR 1999 UNITED STATES, vol. 96, no. 5, 2 March 1999 (1999-03-02), pages 2508-2513, XP002469783 ISSN: 0027-8424
- POLSTER B M ET AL: "Postnatal brain development and neural cell differentiation modulate mitochondrial Bax and BH3 peptide-induced cytochrome c release" CELL DEATH AND DIFFERENTIATION 01 MAR 2003 UNITED KINGDOM, vol. 10, no. 3, 1 March 2003 (2003-03-01), pages 365-370, XP002469784 ISSN: 1350-9047
- DE SARNO P ET AL: "Muscarinic receptor activation protects cells from apoptotic effects of DNA damage, oxidative stress, and mitochondrial inhibition" JOURNAL OF BIOLOGICAL CHEMISTRY 28 MAR 2003 UNITED STATES, vol. 278, no. 13, 28 March 2003 (2003-03-28), pages 11086-11093, XP002469785 ISSN: 0021-9258
- ZHU Y ET AL: "Stimulation of beta2-adrenoceptors inhibits #apoptosis# in rat brain after transient forebrain ischemia" JOURNAL OF CEREBRAL BLOOD FLOW AND METABOLISM, RAVEN PRESS, LTD., NEW YORK, NY, US, vol. 18, no. 9, 2 September 1998 (1998-09-02), pages 1032-1039, XP002098743 ISSN: 0271-678X
- NONAKA S ET AL: "Lithium protects rat cerebellar granule cells against apoptosis induced by anticonvulsants, phenytoin and carbamazepine" JOURNAL OF PHARMACOLOGY AND EXPERIMENTAL THERAPEUTICS 1998 UNITED STATES, vol. 286, no. 1, 1998, pages 539-547, XP002469786 ISSN: 0022-3565
- ROWE M K ET AL: "Lithium neuroprotection: Molecular mechanisms and clinical implications" EXPERT REVIEWS IN MOLECULAR MEDICINE 2004 UNITED KINGDOM, vol. 6, no. 21, 2004, page 18p, XP008088820 ISSN: 1462-3994 1462-3994
- OLNEY J.W. ET AL.: 'Do Pediatric Drugs Cause Developing Neurons to Commit Suicide?' TRENDS IN PHARMACEUTICAL SCIENCES vol. 25, no. 3, March 2004, pages 135 - 139, XP004493460
- BITTIGAU P. ET AL.: 'Antiepileptic Drugs and Apoptotic Neurodegeneration in the Developing Brain' PROC. NATL. ACAD. SCI. USA vol. 99, no. 23, November 2002, pages 15089 - 15094, XP003003654
- JEVTOVIC-TODOROVIC V. ET AL.: 'Early Exposure to Common Anesthetic Agents Causes Widespread Neurodegeneration in the Developing Rat Brain and Persistent Learning Deficits' JOURNAL OF NEUROSCIENCE vol. 23, no. 3, February 2003, pages 876 - 882, XP002353922
- STRAIKO MEGAN M W ET AL: "Lithium Protects against Anesthesia-induced Developmental Neuroapoptosis", ANESTHESIOLOGY (HAGERSTOWN), vol. 110, no. 4, April 2009 (2009-04), pages 862-868, ISSN: 0003-3022
- YOUNG C ET AL: "Ethanol causes and lithium prevents neuroapoptosis and suppression of pERK in the infant mouse brain", NEUROBIOLOGY OF DISEASE 200809 US LNKD- DOI:10.1016/J.NBD.2008.05.009, vol. 31, no. 3, September 2008 (2008-09), pages 355-360, ISSN: 0969-9961
- SANDERS R D ET AL: "Dexmedetomidine attenuates isoflurane-induced neurocognitive impairment in neonatal rats", ANESTHESIOLOGY 2009 LIPPINCOTT WILLIAMS AND WILKINS USA LNKD- DOI:10.1097/ALN.0B013E31819DAEDD, vol. 110, no. 5, May 2009 (2009-05), pages 1077-1085, ISSN: 0003-3022

## Description

### BACKGROUND OF THE INVENTION

This invention relates to medicine, neurology, and anesthesiology, and especially pertains to obstetric and pediatric medicine, involving fetuses, infants, and pregnant mothers.

When various parts of the body are being formed during embryonic or fetal development, a surplus number of cells are initially provided for that part of the body; then, in the course of normal development, many of these cells are deleted, by a process called "apoptosis". That term was coined by Kerr and colleagues (Kerr et al., 1972) more than three decades ago, to refer to several interrelated processes by which biological organisms control cell numbers.

There is an ideal number of cells that make up various organs or limbs, in the body of any human or other animal. For optimal health, the body tries to maintain cell numbers in each organ or limb at that ideal level. Most tissues in the body are made up of cells that continually undergo a natural process of replacing cells as they become aged and no longer vigorous. When an aging cell dies, a neighboring cell of the same kind (or in some cases a specialized precursor cell) undergoes cell division, to form a new cell to replace the one that died. This process normally operates in a balanced manner, so that cell numbers for each tissue or organ are maintained at an ideal level.

However, under some circumstances, the cell death process can exceed the rate of replacement. If this occurs and is sustained over time, the affected organ can become impaired or dysfunctional. For example, if the liver is infected by a viral hepatitis that kills large numbers of cells and prevents them from regenerating, the person can suffer serious liver damage, and may die unless he/she receives a liver transplant.

In the opposite direction, it is also possible for the rate of cell reproduction to escape its normal controls, and begin to outrun the rate of cell death. This condition usually results in cancers, benign tumors, or similar problems.

Thus, apoptosis, a process by which cells actively commit suicide, can serve very important functions in maintaining cell numbers at ideal levels, in the organs and connective tissues of an adult animal.

Apoptosis also plays crucially important roles in the development and growth of an embryo or fetus. When various parts of the body are being formed during embryonic life, a surplus number of cells is usually provided, for most parts of the body. Then, in the course of normal development, some of those cells are killed and deleted by apoptosis.

For example, when a human hand or foot is being formed, the hand or foot initially has the appearance of a webbed structure, like a duck's or frog's foot; then, by apoptotic death and deletion of the cells that make up the webbing, the extremity is shaped into a normal-appearing hand or foot, with separate fingers and toes. In a similar manner, the valves in and around the heart are initially formed as simple tubes. Portions of those tubes then dissolve, through a process of cellular apoptosis, leaving behind flaps with specific shapes and connections, which make the valves functional.

Thus, controlled processes of apoptosis play major roles in normal development of various parts of the body. However, the extent to which apoptosis is involved in shaping an organ varies considerably from one organ to another, and for some organs the role of apoptosis involves factors that remain unresolved mysteries.

Apoptosis plays a major role in the development and growth of the immature brain. In a developing mammalian brain, surplus and redundant neurons are initially created, then some of those neurons are killed, and deleted, by apoptotic mechanisms that are comparable to a gardener pruning unwanted shoots and branches from a plant.

Based on recent research, it is becoming clear that this natural process of programmed neuronal death, in immature brains, can be accelerated and increased by certain types of external factors, in ways that can inflict permanent pathological damage on an immature brain. For example, in a series of recent studies (reviewed in Olney et al., 2004a), John Olney and his colleagues have discovered that during "synaptogenesis" (i.e., a period in fetal and neonatal life when neurons are growing rapidly and establishing synaptic connections with one another), neurons are prone to commit suicide at abnormally high rates, if something interferes with the normal processes by which the neurons create, activate, and evaluate synaptic junctions with other developing neurons. The period of synaptogenesis, also known as the brain growth spurt period, occurs in various species at different times relative to the time of birth (Dobbings and Sands, 1979). In rats and mice, it continues, after birth, for roughly 3 weeks of neonatal life, whereas in humans it begins in the second trimester of pregnancy and lasts until 3 to 4 years after birth. Thus, data generated in postnatal rodents have relevance to both the prenatal (fetal) and postnatal periods in humans. The interference that can cause immature neurons to commit suicide may be either environmental or genetic in origin, and can be very simple, in nature.

For example, various drugs that suppress neuronal activity can trigger neuronal suicide in the immature rodent brain. These types of drugs usually impart an anesthetic, sedating, anti-convulsant, or similar effect in either the immature or adult brain. Exposure of neurons in the adult brain to these drugs, even for many hours, does not cause adult neurons to commit suicide, but exposure of immature neurons for only several hours does cause neuronal suicide. If humans are as sensitive as rodents to this neurotoxic effect, this would signify that treating either human infants or pregnant women with these drugs poses risk of triggering neuronal suicide in the immature brain. Treating pregnant women entails risk because many of these drugs freely penetrate placental barriers to gain access from the maternal blood to the fetal circulation, which delivers the drug to the fetal brain.

Alternately, various genetic mutations have been created and tested in mice, which cause certain neurotransmitter or neurotrophic substances to be dysfunctional in regulating neuronal activity. Since the proper construction, activation, and operation of neurons and synaptic activity require each and all of numerous different proteins and enzymes to function properly, the deletion of even a single gene that is involved in the process typically creates a "break in the chain", which will hinder synaptogenesis. Regardless of which particular form or manifestation the hindrance may take, the simple fact that synaptogenesis is indeed being hindered, and suppressed, will cause neurons in the brain to commit suicide in abnormally large numbers, during the period of synaptogenesis.

When nerve cells die, they are not replaced. Neurons in most regions of the developing central nervous system (CNS) simply do not have the regenerative capacity to form new nerve cells, to replace neighboring cells that have died. Once a neuron dies, it is deleted from the nervous system forever, and not replaced.

As a result, if an increased rate of apoptotic cell death occurs in the immature brain, it will have a much more lasting and profound impact on an animal, than a temporary increase in apoptotic cell death in other tissues of the body. If neurons are deleted in abnormally large numbers from an individual's brain, during fetal development, it will permanently impair the ability of that individual to function normally, and the extent and severity of the impairment will be generally proportional to the numbers of neurons that were abnormally deleted, during that developmental stage in the immature brain.

In the central nervous system (CNS , i.e., the brain, spinal cord, and retina), the role of apoptosis in normal development is poorly understood. It is known that during normal development of the CNS, a certain percentage of neurons will die by apoptosis; however, scientists have not had reliable methods for detecting neuro-apoptosis, or for measuring the varying rates at which it occurs, in various regions of the developing CNS. As will be described below, Olney and colleagues, using methods that have only recently become available, have made important new inroads in understanding the role of apoptosis in normal development, and they recently have discovered ways in which the rate of neuro-apoptosis can become abnormally increased, thereby causing excessive and pathological deletions of neurons from immature brains.

Before describing the recent findings of Olney and colleagues, it is necessary to explain that there is a great deal of confusion in the medical literature, regarding the role of apoptosis in diseases of the CNS. In the human CNS, very early during development, progenitor cells divide and multiply and their progeny differentiate into neurons. Once that has happened, the process of neuronal generation stops and, with very minor exceptions, there are no additional neurons formed in the brain for the remainder of a person's life.

Because neurons in the mature brain do not have the capacity to generate new neurons, they also do not have a pathological tendency to cause cancer. It is true that cancer sometimes occurs in the brain or spinal tissue; however, except in very rare cases, these are not tumors of nerve cells; they are tumors of glial cells, which provide a supportive matrix in the brain. Glial cells cannot send or receive nerve signals; however, they do have regenerative potential, and therefore, they are susceptible to forming cancer.

Mature neurons do not divide and multiply their numbers, so there is no provision among neuronal cells for an apoptotic process to keep neuronal multiplication in check. In the literature, there has been much speculation that apoptosis may play a role in various neurodegenerative diseases of the adult CNS; however, evidence to support that speculation is not very strong. Therefore, at the present time, it is uncertain whether apoptosis is a cell death mechanism that plays a significant role in any neurodegenerative diseases of the adult CNS.

Another factor that has also contributed to confusion, misinterpretation, and misunderstanding in the medical literature centers on the relationship between two very different processes by which nerve cells die in the developing CNS - apoptotic cell death, and excitotoxic cell death.

As already mentioned, apoptotic nerve cell death occurs naturally to some extent in the developing CNS; it also can be induced to occur at a markedly increased and pathological rate, by administering certain types of drugs, and under some types of abnormal circumstances. Excitotoxic nerve cell death does not occur as a natural phenomenon during development, but it can be induced to occur to a minor, or sometimes major (and even catastrophio) extent, in the immature brain. The circumstances that induce apoptotic neuronal death, versus excitotoxic neuronal death, are very different; indeed, in a number of respects, they are the opposite from one another.

Excitotoxic cell death occurs when an excitatory neurotransmitter (glutamate) excessively activates synaptic receptors, through which it normally transmits messages from one nerve cell to another. Because of certain cellular and physiological factors, excessive stimulation of excitatory receptors on CNS neurons, to a point where the neurons become exhausted and badly depleted, can literally kill neurons in the brain or spinal cord, hence the term, "excitotoxic cell death". Circumstances under which excitotoxic cell death can occur in neonates or infants include hypoxia/ischemia (inadequate oxygen or blood supply, sometimes referred to as perinatal asphyxia, which can occur during birth, for example, if the umbilical cord becomes wrapped tightly around the neck, or if the mother suffers from shock, severe blood loss, or other problems), hypoglycemia, status epilepticus, and traumatic brain injury.

By contrast, apoptotic cell death in the developing brain is triggered, not by *excessive* excitatory stimulation, but instead by *insufficient* excitatory stimulation. More broadly speaking, it occurs under circumstances of stimulus deprivation to a point that leads to neuronal inactivation, as discussed in more detail below.

A major reason for confusion in this field is that in immature brains, certain types of conditions (including (hypoxia/ischemia, hypoglycemia, status epilepticus, and traumatic brain injury, as listed above) can cause both excitotoxic and apoptotic nerve cell death, and most researchers have not used methods appropriate for differentiating between those two processes. Rather, they have tended to lump together those two very different types of neuronal death, as if they were the same process. Some researchers even use terms such as, "excitotoxic apoptosis", a misnomer that reflects the confused state of affairs in this area of research.

Olney and colleagues have described methods and criteria for distinguishing between excitotoxic versus apoptotic cell death in immature brains, and they have clarified the relationship between the two processes. For example, they have shown that either hypoxia/ischemia or head trauma can trigger, in an infant rat brain, both an acute type of excitotoxic neuronal death (which is completed within 4 hrs) and a delayed type of apoptotic cell death (which evolves over 8 to 24 hrs). They also have shown that those two very different processes can be distinguished quite readily, not just because they occur at separate locations and over separate times, but also by using tools such as ultrastructural evaluation, which clearly show that the types and sequences of morphological damage and alteration that characterize excitotoxic degeneration are clearly different from other types of changes that characterize apoptosis.

It is especially important to recognize and understand the distinctive characteristics of those two different types of neuronal death processes, because it is very likely that prophylactic and treatment methods that can prevent or minimize and control one type of cell death, will be ineffective in preventing or reducing the other type of cell death. Moreover, since the apoptotic cell death process has a delayed and more protracted time course, physicians and researchers need to understand that there may be, in at least some apoptotic cell death processes, a longer window of opportunity for therapeutic intervention than is the case for excitotoxic cell death.

To explain further the concept of deprivation leading to apoptosis, it is instructive to examine what happens in head trauma. A concussive blow creates a severe impact, at a certain place on the skull. Inside the skull, immediately adjacent to the impact, an area of excitotoxic neurodegeneration will occur, acutely and rapidly, leading to end-stage cell death within about 2 to 4 hours.

Subsequently, beginning at about 8 hours, signs of apoptotic cell death will begin to emerge, often at sites that are remote from the brain regions directly beneath the skull at the point of impact. This apoptotic cell death process evolves, with different degrees of intensity in various brain regions, over a period of approximately 8 to 24 hrs.

The extent, and the patterns, of apoptotic neuronal death that will occur, at brain regions that are remote from the location of the direct brain tissue injury, have been discovered, by Olney and colleagues, to depend heavily on whether the immature brain is passing through a developmental stage called "synaptogenesis" (also known as the "brain growth spurt" period).

During this developmental period of synaptogenesis, all neurons in the brain are receiving synaptic inputs from distant neurons, and they also are sending out long fibers to make synaptic contacts with distant neurons. The initial traumatic event triggers a wave of excitotoxicity, which will kill nerve cells that are "subjacent" to the impact site. A type of semi-liquid "wave" that travels through the brain tissue can also cause physical strain and sheering forces, which can injure and even sever some of the axonal fibers through which neurons in one brain region make synaptic contacts with neurons in other regions. As a result, various neurons that are remote from the direct impact site will be deprived of incoming synaptic signals, and/or they will be deprived of synaptic targets. Accordingly, if excitotoxic destruction of neurons, and physical destruction of fiber connections, causes neurons in other brain regions to be deprived of both synaptic inputs, and synaptic targets, the remote neurons will sense that they are failing to perform their synaptic mission. That failure will trigger an apoptotic response, in which the remote neurons will commit suicide.

As will be discussed in more detail below, various drugs that can inhibit neuronal activity can also cause neurons in an immature brain to be placed in an "activity-deprived" condition, even when the brain has suffered no physical or mechanical damage. Recent research has shown that developing neurons in an immature brain require a certain level of nerve signal activity, in order to meet what can be regarded as a "synaptic connectivity schedule". If neurons in an immature brain are suppressed by such drugs, to abnormally low levels of activity, they will not meet their schedules. If they sense that they are failing to meet their schedule, they interpret that as an indicator that they apparently belong in the set of neurons that simply failed to be chosen and woven into the interactive "fabric" of brain activity. As a direct result of that lack of activity, certain proteins will activate certain signals, and those neurons will be killed, by the same process of apoptosis that is actively pruning out and eliminating other unwanted and unused neurons in a process that, in a healthy brain, brings the surplus number of starting neurons down to a proper long-term level.

Apoptosis is recently becoming recognized as a disease mechanism in the developing nervous system. In contrast to the uncertain situation in adult neurodegenerative diseases, it is clear from the research data, and from the conclusions that are supported by those data, that apoptosis decidedly plays an important and even major role in a number of entirely natural diseases that afflict immature brains and spinal cords. It also is becoming clear that apoptosis, arising from nerve signal deprivation caused by a pregnant female's intake of ethanol (i.e., ethyl alcohol, the intoxicant in beer, wine, and liquor) is the major causative factor in the type of brain damage known as "fetal alcohol syndrome".

In addition, very recent research has created a growing body of evidence that apoptotic damage to immature brains can be caused by "iatrogenic" situations, in which the immature brain is exposed, during medical treatments for other conditions, to drugs that can trigger neuroapoptosis.

The data and conclusion that certain types of drugs that are commonly used as sedatives, anesthetics or anticonvulsants in pediatric and obstetric medicine, can trigger developmental neuroapoptosis, are only very recently becoming recognized. Olney et al. recently have shown that exposure of the immature CNS to any of several classes of pharmacological agents, during the rapid brain growth period of synaptogenesis, causes developing neurons in the brain, spinal cord, and retina to commit suicide (i.e., to die by apoptosis). Drugs that will trigger neuroapoptosis in these fetal and neonatal animal models include three major categories of compounds, all of which have various known effects and uses. Those three categories are:
(1) drugs that block receptors for an important excitatory transmitter system, known as the "NMDA" subclass of glutamate receptor (NMDA refers to N-methyl-D-aspartate, a probe drug that was discovered to powerfully and selectively activate those particular receptors); stimulation of NMDA receptors by the excitatory transmitter, glutamate, increases neuronal activity, and drugs that block these receptors (known as NMDA antagonist drugs) suppress neuronal activity.
(2) drugs that activate receptors for an important inhibitory transmitter system, known as the GABA_{A} receptor system (GABA refers to gamma-amino-butyric acid, which is the predominant inhibitory transmitter in the mammalian brain. Activation of GABA inhibitory receptors suppresses neuronal activity (compare with blockade of NMDA excitatory receptors, which also suppresses neuronal activity).
(3) drugs that block "voltage-gated" sodium ion channels, which are crucial in allowing neurons to undergo the ionic flows and chemical surges that create firing events and signal transmissions. Blocking these sodium ion channels also suppresses neuronal activity.

It also should be noted that ethanol, the culprit in fetal alcohol syndrome, strongly suppresses nerve signal transmissions, by two separate mechanisms: (i) it blocks NMDA excitatory receptors; and, (ii) it activates GABA_{A} inhibitory receptors.

These neuroapoptosis findings in laboratory animals, which have been recently published by Olney et al. (e.g., Olney et al., 2004a, and 2004b, and Jevtovic-Todorovic et al., 2003), have considerable significance in a public health context, and these reports have generated substantial controversy in the medical community, because a number of drugs that are commonly used in obstetric and pediatric medicine (mainly as sedatives, anxiolytics, anticonvulsants, or anesthetics) fall squarely into the categories listed above. The status of this controversy at the time of this writing is that Olney and colleagues have raised the question whether pediatric drugs cause immature neurons to commit suicide in the developing human brain, and critics have responded by arguing (e.g., Anand and Soriano, 2004; Soriano et al., 2005) that it is invalid to extrapolate data from rodent experiments to the human situation, and they have emphatically expressed the view that it would be totally inappropriate to introduce any changes in the practice of human anesthesia based on such animal experimentation. Indeed, Soriano et al. (2005) have argued that if such changes were introduced it "may even be deleterious to long term neurologic outcome." Other critics have claimed (McClaine et al., 2005) that the findings of Olney et al., that anesthetic drugs kill neurons in the immature rat and mouse brain, cannot be reproduced in sheep, which they refer to as a higher order species. They suggest that the Olney et al. findings can be explained either by a species difference or by failure of Olney et al. to properly control cardiorespiratory function when they anesthetized their immature rodents. The gist of this issue, which was also raised by Soriano et al., (2005), is that because of the small size of immature rodents, it is impossible to intubate them and control their respirations while they are anesthetized. Therefore, critics claim that these animals have sustained brain damage, not due to the anesthetic drug, but due to lack of oxygen supply to the brain. McClaine et al. performed their experiments on pregnant sheep, which are large enough to be maintained on a mechanical respirator that automatically controls respirations throughout the anesthesia period. Since they have maintained adequate control over respiratory function in their sheep experiments, similar to the control exercised in human anesthesia, they argue that their experiments are clinically relevant, whereas the Olney et al. experiments are clinically irrelevant. On the basis of the McClaine et al. (2005) findings, published recently in an article coauthored by 11 anesthesiologists and surgeons, it was concluded that "these results .....corroborate the presumed safety of inhalational anesthetic use during pregnancy." Thus, at the present time, the medical community is expressing the view that the Olney et al findings have no relevance beyond the realm of rodents, and even in rodents, the brain damage reported may be attributable to hypoxia/ischemia and not to an effect of anesthetic drugs.

While the medical establishment does not currently consider the Olney et al. findings to have direct relevance to human medicine, they do readily acknowledge that all anesthetic drugs used in obstetric or pediatric surgery are either NMDA antagonists (i.e., agents that block excitatory neurotransmission through NMDA glutamate receptors), or GABA_{A} agonists (i.e., agents that inhibit nerve signal transmission through GABA_{A} receptors). In addition, they do acknowledge that typically, these agents are used in combination, so that the brain of a neonate or young infant who requires surgery (for example, to repair a congenital heart defect) is likely to be exposed to two or more GABA_{A} agonists and NMDA antagonists, at the same time, during the surgery.

In addition, it is well recognized that many anticonvulsant drugs that are typically used to treat pregnant women or infants who suffer from epileptic seizures are either GABA_{A} agonists, or sodium channel blockers.

Ethanol, which has both NMDA antagonist and GABAₐ agonist activity as noted above, triggers especially severe neuroapoptosis responses in immature brains (Ikonomidou et al., 2000). It has long been known that ethanol, if ingested by a pregnant mother either chronically, or even just in a small number of "binge drinking" episodes, has toxic effects on the brain of the fetus she is carrying; this condition is widely referred to as fetal alcohol syndrome (FAS). The recent findings by Olney and colleagues have elucidated the mechanism underlying that type of neurotoxic damage inflicted by ethanol on immature brains. The known vulnerability of the immature human brain to apoptogenic damage by ethanol, together with evidence that anesthetic and anticonvulsant drugs trigger neuroapoptosis in immature animal brains by ethanol-like mechanisms (i.e., through NMDA receptor suppression, and/or GABA_{A} receptor activation) lends considerable credibility to the assumption and conclusion that anesthetic and anticonvulsant drugs very likely are indeed triggering unwanted, unnatural, and pathological levels of neuroapoptosis, in the immature and developing brains of human neonates and infants who require surgery, anticonvulsant drugs, or similar medical treatments.

There is also major public health significance in the observation that apoptotic neuronal death in neonates or infants plays an important role in the brain damage that occurs after acute brain injury in a neonate or infant (such as a hypoxic/ischemic crisis, head trauma, etc.). The reasons that support this conclusion include:
1. Acute injury episodes, in neonates and infants, frequently become sources of lifelong developmental disabilities; for example, nearly all cases of cerebral palsy, a lifelong disability, arise from just a few minutes of oxygen deprivation during birth;
2. Animal tests have documented that in both hypoxia/ischemia and head trauma, the magnitude of cell loss, due to delayed neuroapoptosis, is usually far greater than the magnitude of cell loss that arises directly from the initial excitotoxic insult; and,
3. Because the neuroapoptosis response occurs on a delayed time schedule, there will be a longer window of opportunity for therapeutic intervention. For example, if an infant is brought to an emergency room very soon after head trauma, the acute excitotoxic neurodegeneration will be occurring in this early period (it completely runs its course in the first 4 hrs after head trauma), but the apoptotic delayed response, which results from activity deprivation, does not begin to manifest until approximately 8 hours, at the earliest. Therefore, if the infant arrives in the emergency room 1 hour following head trauma, there is still a period of 7 hours for a neuroprotective drug to work before apoptotic degeneration would be expected to ensue. This provides an ample time window for the neuroprotective drug to "precondition" the synaptically deprived neurons, so that they will be bolstered against undergoing apoptotic degeneration. Thus, much of the delayed apoptotic degeneration may be preventable, and this represents the majority of neurons that would ordinarily die after such a traumatic event.

It should be noted that the greatest vulnerability of a neonate or infant, to developmental neuroapoptosis caused by surgical anesthetics or similar drugs, or following an acute head injury or insult, is effectively limited, or at least heavily focused, on the period of synaptogenesis. The time window during which the immature brain is most highly sensitive to damage caused by apoptogenic drugs coincides with the period of synaptogenesis, also known as the brain growth spurt period. This period occurs in different species at different times, relative to birth. In rodents, it occurs mainly postnatally (for example, in rats, it occurs during the first two or three weeks after birth). In humans, it begins in mid-gestation, and it continues (at varying rates) until about three years after birth (Dobbing and Sands, 1979).

As mentioned above, "synaptogenesis" refers to the period during which neurons establish functioning contacts (known as synapses, or synaptic junctions) with one another. It is through these synaptic junctions that nerve impulses are transmitted, and during the period of synaptogenesis, billions of such contacts are established. This is a period of rapid brain growth because, in order to receive incoming synaptic connections, each immature neuron expands its surface area by growing new branches, much like a young tree growing new branches.

All of the drugs that have been shown to trigger neuroapoptosis, in animals, during the synaptogenesis period appear to have one property in common: they abnormally suppress neuronal activity. It is known that nerve cells are programmed to kill themselves if, during development, their biological clock-like sensing mechanisms detect that they are failing to make synaptic contacts in a timely manner, and in the proper sequence. A drug that abnormally suppresses neuronal activity will disrupt the "synchrony" of the synaptogenesis process (i.e., suppressed neurons will become "out of phase" with other neurons that were not directly affected by the drug). This can cause large numbers of neurons to sense that they are out of synchrony, and effectively were not chosen for inclusion, in the synaptogenesis process of brain development. Within the desynchronized neurons, this type of perceived exclusion will automatically generate internal signals that activate the proteins that drive apoptosis. That process of neuroapoptosis, triggered accidentally and unintentionally by drugs that artificially suppress neuronal activity, will culminate in the deaths of neurons that otherwise would have survived and played their proper roles in the developing brain.

### Biochemical Pathways that Mediate Immature Neuroapoptosis

The preceding discussion focuses on general principles. Once those general points have been established, attention must be turned to various specific proteins and signals that become involved in the mechanisms of neuroapoptosis.

It should be noted that apoptosis is a well-known phenomenon, which has been receiving an increasing amount of attention since the early 1970s when Kerr and colleagues (1972) coined the word "apoptosis" and pointed out that this form of cell death plays an important role in many different tissues of the body and under both normal and pathological circumstances. In the ensuing years much has been learned about apoptosis as it occurs in *vitro,* and in many different tissues and organs *in vivo.* One common form of apoptosis prominently involves mitochondria, which are energy-producing organelles contained within all living cells. Very briefly, when various types of "upstream" processes occur, including processes that involve a death-promoting group of proteins (e.g., Bax protein), the mitochondrial membranes become abnormally permeable. When this happens, the mitochondria begin releasing a signaling protein called cytochrome c, sometimes called the "cell death signal". Increased membrane permeability and release of cytochrome c create a transition, and the steps that occur after cytochrome c has been released can be regarded as "downstream" steps, culminating in the activation of a protease enzyme called caspase-3 which, together with other enzymes that it activates, degrade the cell into fragments that are ingested and digested by cells known as phagocytes.

Apoptosis in the CNS is not as well understood as in other tissues of the body because there have not been any good in vivo models for studying the phenomenon. However, the animal models recently developed by Olney and colleagues for inducing neuroapoptosis throughout many regions of the immature animal brain are now providing an opportunity to study the mechanisms underlying in vivo CNS apoptosis in detail. Using these models, Olney and colleagues have determined (Young et al., 2003) that this apoptosis process conforms to the mitochondrial model described above. It is a Bax-dependent process, involving translocation of Bax protein, normally not in contact with mitochondria, to the external surfaces of the mitochondrial membranes. Bax protein disrupts membrane integrity(Dikranian et al., 2001) leading to increased membrane permeability and the release of cytochrome c, which triggers the same series of steps referred to above, culminating in activation of caspase-3 (Olney et al., 2002b) and terminal degradation of the cell (Young et al., 2005). Thus, in this example of CNS apoptosis, as in many other apoptosis processes, mitochondria play a key role in defining the transition from a living cell to one that is irreversibly committed to cell death. After Bax protein has altered the integrity of the mitochondrial membrane, the cell death cascade has begun and even if interrupted, the cell is already doomed to die.

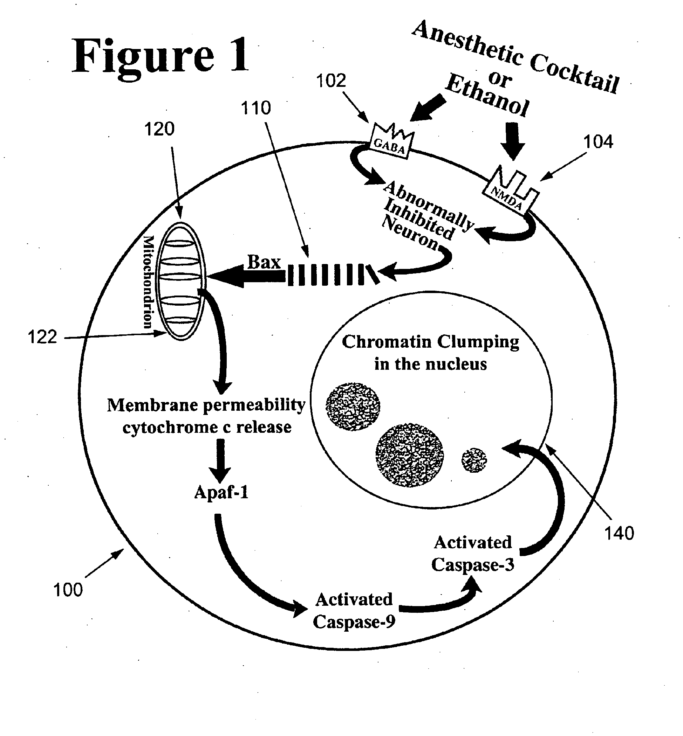

These biochemical steps in the CNS apoptosis cell death process are presented schematically in Fig. 1.

Accordingly, while apoptosis is relatively well-understood in many tissues of the body, we are just beginning to develop an understanding of this cell death process as it affects neurons in the developing CNS.

More importantly, there is no indication in the medical literature that medical scientists or authoritative medical organizations are recognizing developmental neuro-aoptosis that can be induced by drugs that are commonly used in pediatric and obstetric medicine as a problem that requires corrective measures to prevent such drugs from causing harm to human fetuses and infants.

In some prior art references, however, the occurrence of pathological neuroapoptosis in relation to drug treatment has been addressed.

Accordingly, WO 2006/018655 discloses the use of a combination of the volatile anesthetic xenon in conjunction with another volatile anesthetic, e.g. sevoflurane, in neonates, where xenon is used to protect against the anesthetic-induced neuroapoptosis. The anesthetic and anti-apoptogenic functions of xenon are difficult to dissect, however, thus rendering the process of assessment of its neuroprotection vs. anesthetic effects unnecessary cumbersome.

The anti-apoptogenic agent melatonin was found to protect against pathological neuroapoptosis in immature rats undergoing anesthesia, where melatonin was administered subcutaneously protect against neuroapoptosis triggered by a cocktail of anesthetic drugs (Beals et al, 2003). The effects of a melatonin pretreatment were only modest in the experimental setup; moreover, the antiapoptotic effects of melatonin in general are not undisputed (Harms et al. 2000).

Lithium was found to act as an antiapoptotic agent in the context of apoptosis induced by anti-convulsants such as phenytoin and carbamazepine in a tissue culture model (Nonaka et al., 1998), but only in a dose range well above the plasma levels considered therapeutic in humans.

Accordingly, one object of this invention and application is to disclose what is currently known about the risks that certain classes of drugs (including anesthetics used in obstetric or pediatric surgery) pose of inflicting permanent brain damage on fetuses, neonates, and infants, by mechanisms that involve: (i) disruption of essential processes of synaptogenesis, within immature brains, (ii) the triggering of pathological neuroapoptosis, caused by the disruption of synaptogenesis within immature brains.

Another object of this invention and application is to disclose certain already-known agents that can be used to provide surgical anesthesia and various other medical treatments for fetuses, neonates, or infants, without substantially disrupting the essential processes of synaptogenesis within immature brains, and without creating unacceptably high risks of inflicting permanent brain damage on fetuses, neonates, or infants who need surgical or medical intervention.

Another object of this invention and application is to disclose testing criteria that can be used to identify other, additional agents that can provide effective medical treatments for fetuses, neonates, or infants who need such assistance, but without disrupting the critical and essential processes of synaptogenesis in immature brains, and without creating unacceptably high risks of inflicting permanent brain damage on such patients.

These and other objects of the invention will become more apparent through the following summary, description, and examples.

In the fetal brains of mammals, a surplus of neurons is initially created. Many of those neurons are later pruned and deleted, if they do not become fully integrated into functioning neural networks. That form of neuronal killing and deletion is controlled by a "programmed cell death" process called apoptosis, which in turn is controlled by mitochondria, which are organelles that are present in multiple copies inside each neuron. During a developmental stage called synaptogenesis, immature neurons become exceptionally sensitive to "activity deprivation", and can trigger their own deaths, through apoptosis, if they are deprived, for even a short period of time (such as a few hours) of signals that otherwise would indicate active engagement and involvement in the developing brain. This type of "activity deprivation", which will trigger pathological neuronal death, leading to permanent brain damage and mental impairments, can result from exposure to anesthetic drugs, such as surgical anesthetics that are commonly used on neonates or infants who may need, for example, surgical repair of a heart defect.

That type of pathological neuroapoptosis, and the permanent brain damage it causes, is largely preventable, using the composition described herein.

With regard to surgical anesthesia involving fetuses, neonates, or infants, improved anesthetic regimens are disclosed that will avoid or minimize the apoptotic destruction cascade.

The invention arises from several observations and insights, which include the following:
1. Survival of developing neurons is regulated by several intracellular signaling pathways, all of which have a common denominator. They all regulate cell survival by influencing the homeostatic balance between death-promoting (e.g., Bax and Bak) and survival-promoting (e.g., Bcl-2 and Bcl-xL) members of a Bcl-2 multidomain family of proteins. Under normal circumstances, the balance is maintained in favor of the survival-promoting factors, and the death-promoting factors are held in check. The above mentioned deprivation conditions cause changes in intracellular signaling pathways that shift the balance and allow a death promoting factor (Bax protein) to become dominant and trigger cell suicide.
2. One mechanism by which "activity deprivation" triggers neuroapoptosis involves inactivation of the ERK MAP-kinase intracellular signaling pathway. This signifies that pharmacological treatments that have an activating effect on the ERK MAP-kinase pathway can counteract apoptotic signals triggered by a period of activity deprivation, and can help prevent early apoptotic signals from being carried all the way through in ways that would lead to pathological neuronal destruction.
3. Lithium is a promising candidate for achieving the purposes of this invention, since it has an activating effect on the ERK MAP-kinase pathway.
4. Another mechanism by which deprivation conditions trigger neuroapoptosis is by inactivating the PI3K/AKT intracellular signaling pathway. This signifies that pharmacological treatments that have an activating effect on the PI3K/AKT pathway can counteract apoptotic signals triggered by a period of activity deprivation, and can help prevent early apoptotic signals from being carried all the way through in ways that would lead to pathological neuronal destruction.
5. PKA and PKC signaling pathways are known to have a regulatory influence on the Bcl-2 family of death/survival proteins, which signifies that pharmacological agents that act on the PKA and PKC signaling pathways are promising candidates for preventing immature neurons from committing suicide, if subjected to a period of activity deprivation.

### BRIEF DESCRIPTION OF THE DRAWINGS

FIGURE 1 is a schematic depiction of an immature neuron during the developmental stage known as "synaptogenesis", indicating several major steps that will occur if apoptosis is triggered by a period of "activity deprivation". While passing through that vulnerable stage, if neuronal activity is artificially inhibited by ethanol, a surgical anesthetic, or a similar drug, a series of "upstream" events will occur, culminating in activation of Bax signalling proteins, which will indicate to the mitochondria in the cell that the neuron is a surplus neuron that has not been connected into the neuronal networks of the brain, and it should be destroyed and deleted. The Bax signals will cause the mitochondrial membane to become permeable, allowing the release of a signaling protein called cytochrome C, also called the "cell death signal". The release of cytochrome C triggers a series of "downstream" events involving Apaf-1, caspase-9, and caspase 3 proteins, which drive the rapid death and destruction of the neuron, so that its building blocks can be recycled and used to form other non-neuronal cells in the growing fetus or infant.

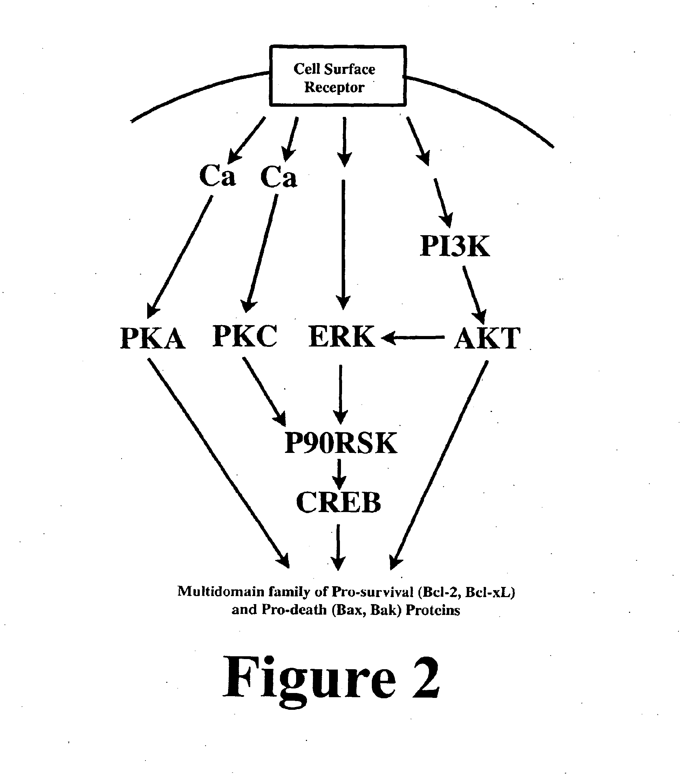

FIGURE 2 depicts a number of signaling pathways that participate in determining whether a neuron in a fetal or infant brain will live, or be destroyed and deleted by apoptosis. These signaling pathways influence the homeostatic balance between the "Bcl" family of proteins (which hold apoptosis in check, allowing a neuron to live and become part of the brain), and the Bax and Bak family of proteins (which tip the balance in favor of apoptosis and cell destruction, if an absence of neuronal activity indicates that some particular neuron is a surplus neuron that did not become connected in the neuronal networks being formed in the brain). If one or more of these signaling pathways are artificially suppressed, such as by ethanol, a surgical anesthetic, or a similar drug or by head injury or excitotoxic crisis in some portion of the brain, the balance will be shifted in favor of the pro-death Bax and Bak signaling proteins. A pharmaceutical intervention that activates one or more of these pathways will be needed, to prevent pathological neuroapoptosis from killing neurons that should have been spared and allowed to live.

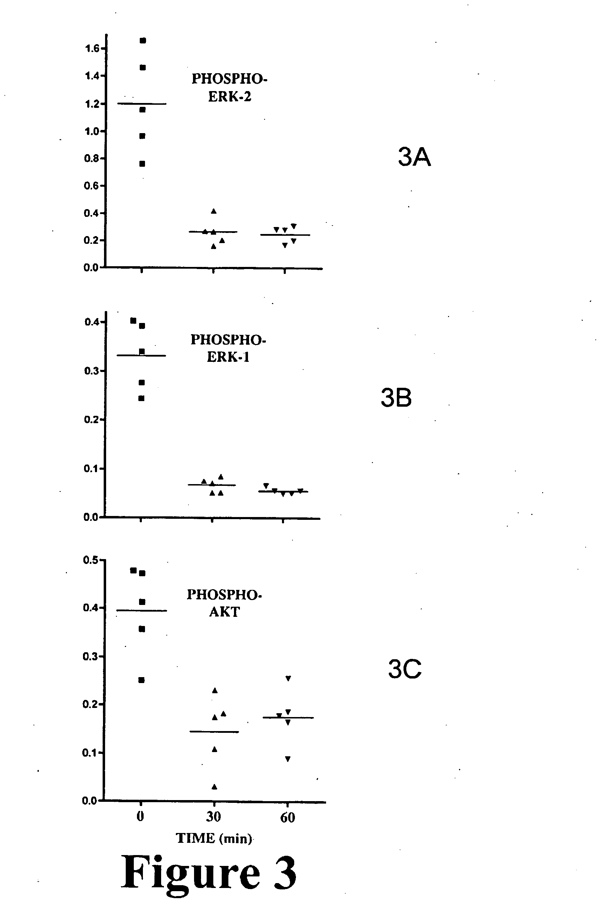

FIGURE 3 (which comprises graphs 3A, 3B, and 3C) shows how ethanol administration in immature mice will suppress activity in three of the signaling pathways illustrated in FIG. 2.

### DETAILED DESCRIPTION

As summarized above, and as depicted schematically in FIG. 1, pathological forms of neuroapoptosis in the immature brain can be caused by even brief periods of "activity deprivation", if those periods of activity deprivation occur during critical and vulnerable stages of synaptogenesis, when the brain must determine which neurons to keep and which ones to delete: Such periods of "activity deprivation" can be caused by ethanol intake by a pregnant female, or by various types of anti-convulsant, anesthetic, or sedative drugs, such as surgical anesthetics that are used on neonates or infants who may need surgical repair of a congenital defect. Accordingly, FIG. 1 schematically depicts an immature neuron 100 that is being subjected to ethanol intake by the mother, or surgical anesthesia. Either of those two activities will artificially suppress neuronal activity, by acting at GABA and/or NMDA receptors 102 and 104, located on the surface of the immature neuron 100.

Inside neuron 100, a series of events that involves several distinct signaling pathways, represented by a row of dominoes 110 inside neuron 100 (several of these signaling pathways are identified below, and in FIG. 2), will lead to activation of a set of proteins designated as the BAX protein family. When activated, these proteins are translocated from their normal positions, floating in the "cytosol" that fills most of the cell, to the external surfaces of the mitochondria that are contained in the cell. Each neuron holds dozens or hundreds of mitochondria, which are small organelles that are enclosed within their own membranes. These numerous mitochondria are schematically represented in FIG. 1 by a single mitochondrion 120, enclosed within mitochondrial membrane 122.

When the mitochondrial membrane 122 is contacted by activated Bax proteins, it becomes permeable, which allows the release of a signalling protein called Cytochrome C. As mentioned above, Cytochrome C is sometimes called the "cell death signal". The transition to membrane permeability, accompanied and indicated by the release of Cytochrome C, is regarded as a boundary or transition between various "upstream" steps and stages in apoptosis, and various "downstream" stages.

The signaling steps shown in FIG. 2 (and represented by the row of dominoes 110 in FIG. 1) are part of the "upstream" stages of apoptosis. Similarly, activation of the Bax signaling protein, its translocation to mitochondrial membrane 122, and any steps that occur that lead up to the onset of membrane permeability, also are regarded as part of the "upstream" stages of apoptosis. This is an important distinction, because interventions that can target one or more specific steps in these "upstream" stages that lead to apoptosis offer the best candidate mechanisms for pharmaceutical interventions that can halt and arrest the process of apoptosis, even after one or more triggering events have occurred, before any permanent damage is done to the immature neuron.

By contrast, once the mitochondrial membrane 122 has become permeable (as evidenced by the release of Cytochrome C by the mitochondria), the "downstream" stages of apoptosis will commence. This includes the activation of the Apaf-1, caspase-9, and caspase-3 proteins, and the changes that will occur in the neuron's nucleus 140.

It is not conceded herein that it is impossible to intervene and stop one of more of those "downstream" protein activations or activities, in ways that can rescue immature neurons; indeed, such efforts merit evaluation, in view of their potential to help rescue immature neurons and minimize permanent brain damage. However, because of practical factors, it should be recognized that earlier interventions, designed to prevent the activation of Bax proteins and the release of Cytochrome C (the "cell death signal"), appear to offer greater promise for providing useful and efficient interventions.

In particular, it should be recognized that dozens or hundreds of mitochondria, in a single neuron, may be releasing substantial numbers of Cytochrome C molecules in that neuron, and any of those Cytochrome C molecules may be able to relaunch and restart the downstream death cascade once again, even if an initial set of activated Apaf-1, caspase-9, or caspase-3 molecules were blocked and inactivated by a pharmaceutical treatment. Furthermore, even if methods or agents can be identified for blocking some particular step in the downstream pathway, such efforts might lead to undesired outcomes, if they end up rescuing neurons that are severely injured and that might have disruptive influences on neural networks.

Nevertheless, these types of "downstream" interventions are worth studying in animal models, since they might lead to useful therapeutic benefits (as evidenced by behavioral, memory, or similar tests on the animals), and since the data gathered in such research may end up shedding additional light on various processes of neuroapoptosis, which (as noted above) are only poorly understood under the prior art.

As described in greater detail below, and as illustrated in FIGS. 2 and 3, the Applicant has identified certain proteins (including AKT, ERK-1, and ERK-2) in the upstream apoptosis pathways that are markedly altered within 30 minutes after ethanol administration. In this early period, expression of the active (phosphorylated) isoforms of these two proteins is markedly suppressed. Both of these proteins have been identified as proteins that regulate cell survival, by means that appear to require a steady and gradual low-level supply of the phosphorylated isoform. If the gradual production of these active isoforms is hindered or blocked, their absence will contribute to switching the balanced signaling mechanisms from a pro-survival balance (which is manifested by sustained levels of certain proteins, including Bcl-2 and Bcl-xL, as indicated at the bottom of FIG. 2), toward a pro-cell death (which is manifested by increased levels of activated Bax and Bak proteins). Thus, the AKT, ERK-1, and ERK-2 proteins and certain other related proteins, which are part of the upstream steps represented by dominoes 110 in FIG. 1, offer highly promising targets for pharmaceutical intervention using drugs such as described below.

While a number of candidate agents for use in such interventions are already known and available, as listed and described below, it also must be recognized that other such agents can be readily identified, by any of various relatively simple screening processes. For example, a candidate treatment drug can be administered to an infant mouse or rat, at one or more dosages that are believed to fall within or cover a suitable dosage range. At the same time, or shortly before or after such treatment, ethanol or a suitable anesthetic is also administered, at a dosage that will reliably cause a substantial neuroapoptotic response if that type of response is not blocked by an effective intervening anti-apoptosis drug. Four to eight hours later, the animal is sacrificed, and its brain is examined (using methods such as described in the Examples) to see if the neuroapoptosis response was reduced or prevented by the candidate drug.

As described above, , anesthetics that suppress neuronal activity, can place immature neurons in activity-deprived conditions that can trigger apoptosis. Thus, effective prophylaxis will require an approach that prevents the deprived condition from triggering a suicide signal in some or all of the affected neurons. The subject invention utilizes two separate approaches, either of which can prevent the suicide signal from being generated or acted upon in some or all of the neurons that would die if not protected. These approaches are: (1) activation of whole neurons, and (2) activation of signaling pathways within neurons.

### Activation of Whole Neurons

Since neuronal suppression (deactivation) is the condition that can cause immature neurons to commit suicide, one approach for preventing unwanted and pathological neuronal death involves counteracting a deactivation condition with a stimulus that activates neurons. A potential problem with this approach is that an anti-convulsant or anesthetic drug, in order to provide effective therapy, must achieve a substantial degree of neuronal suppression. However, an important consideration is that both anti-convulsant and anesthetic drugs perform their actions in excess. For example, the most important action of NMDA antagonists for surgical anesthesia purposes is their analgesic action, which is largely achieved by blocking NMDA receptors at certain levels of the spinal cord where NMDA receptors receive pain messages from various parts of the body and relay them up to the brain. There are NMDA receptors on neurons distributed widely throughout many other brain regions, pertaining to neural circuits that mediate other functions that are irrelevant to analgesia or anesthesia. If the deactivating action of anesthetic drugs on neurons throughout these other neural circuits can be counteracted by an activating stimulus that does not interfere with the blockade of NMDA receptors in the spinal pain pathways, many neurons can be saved from becoming suicide victims, while the purposes of anesthesia are also being served.

The same is true for GABA drugs. In addition to their actions in circuits relevant to anesthesia, they also have actions in many other circuits that are irrelevant to anesthesia.

Therefore, one approach disclosed herein involves including, in an anesthetic protocol that is to be applied to a neonate or infant, a pharmacological agent that is capable of selectively counteracting the suppressant action of NMDA or GABA drugs, in neuronal circuits that are irrelevant to anesthesia.

In certain brain regions, such as the caudate nucleus, both GABA and NMDA anesthetic drugs trigger a dense pattern of neuroapoptosis, because neurons in this brain , region are well-endowed with both GABA_{A} and NMDA receptors. However, the suppressant actions of such drugs, within the caudate nucleus portion of the brain, are entirely irrelevant to their desired anesthetic or anti-convulsant actions, which are mediated through other circuits.

Pathological apoptosis of immature neurons, when triggered by activity deprivation, involves alterations in the activity states of specific molecular entities that mediate intracellular functions relevant to neuronal division, differentiation, migration, and/or synapse formation. How well these molecular entities perform their functions is a major determinant of whether the neuron will survive or perish (by apoptosis) during the course of development. Thus, these molecular pathways are, in effect, regulators of cell survival. The molecular entities that mediate these processes are primarily proteins, and they are organized in systems that are sometimes referred to as cascades, in which activation of a first molecule in the system causes activation of a second, which then activates a third, and so forth.

These proteins usually are identified by acronyms that arise from descriptive phrases. As examples, MAP kinases (often abbreviated as MAPK's) are mitogen-activated protein kinases; CREB proteins are cyclic AMP (adenosine monophosphate) response element binding proteins; and ERK proteins are extracellular signal related kinase proteins. These acronyms, and the proteins to which they refer, are well-known among those skilled in the art, and are described in numerous articles that discuss various aspects of apoptosis, cancerous cell growth, and other cellular and physiological processes.

### The ERK-MAP-kinase system as a target for modification

Unpublished evidence generated by Olney and colleagues indicates that a MAP kinase cascade plays a central role in mediating neuroapoptosis induced by deprivation circumstances. MAP kinases were originally studied as regulators of mitosis and more recently have been found to be abundantly expressed in the cell bodies and dendrites of post-mitotic neurons, where it is believed they regulate differentiation, synaptogenesis and survival of neurons.

Three MAP kinase pathways have been identified. These are distinct and non-interactive pathways, each of which has a three-tiered system in which activation of a first kinase (MKKK) leads to activation of a second kinase (MKK), which activates a third kinase (MAPK).

In one system that is of particular interest for this invention, the third kinase in that type of cascade is referred to as ERK (i.e., extracellular signal related kinase proteins, as indicated above). The ERK-MAPK cascade is of special interest in relation to deprivation-induced neuroapoptosis, because it is known that activation of the NMDA receptor causes an increase in the activated (phosphorylated) isoform of ERK, which triggers a translocation of ERK to the nucleus, where it indirectly causes phosphorylation of CREB, a transcription factor that can directly or indirectly activate many genes that control protein synthesis.

Some of the proteins that are synthesized via this pathway are members of the multidomain "Bcl" family of proteins, which regulate cell survival ("Bcl" refers to B-cell lymphomas, which were the cell types in which Bcl proteins were first identified). Proteins such as Bcl-xL and Bcl-2 are anti-apoptotic, i.e., they act to prevent apoptosis from occurring, thereby sustaining and protecting a cell. Other proteins, including proteins in the "Bax" and "Bak" families, are pro-apoptotic.

Normally, the pro-apoptotic and anti-apoptotic proteins are maintained in homeostatic balance, by CREB-regulated synthesis of each protein at a rate conducive to an anti-apoptotic balance (i.e., this balance favors and supports cell survival). However, if this balance is upset, and tipped in a pro-apoptosis direction, by a force external to the system (e.g., by the influence of ethanol, anesthetic drugs, or loss of synaptic inputs), the pro-apoptosis members assume control, and an apoptosis signal is generated and acted upon by Bax, an effector protein that translocates to mitochondrial membranes and initiates a cascade of events leading to mitochondrial membrane permeability and release of cytochrome C, which will culminate in cell death.

The two graphs shown as FIGS. 3A and 3B indicate that administration of ethanol (a potent neuronal activity suppressor, since it has both NMDA antagonist and GABA agonist activity) in infant mice, will cause a rapid decrease (within less than 30 minutes) in the activated isoforms of ERK. This signifies that the NMDA-receptor-linked ERK/CREB pathway is shut down by ethanol, in a way that can disrupt the balance of protein factors that regulate cell survival, altering the balance in a way that promote neuroapoptotic cell death. In this case, decreased neuronal activity, believed to be acting mainly through NMDA receptors, contributes to the reduced levels of activity in the ERK/CREB pathway.

A loss of synaptic inputs, from the neurons that were injured by crisis, would be the initiating step that would cause the ERK/CREB pathway to be suppressed. The loss of arriving signals, which will drop off if the signal-generating neurons are severely damaged, would have the effect of silencing excitatory activity that would be mediated by the same NMDA receptors that can be driven into silence by ethanol, anesthetic drugs, etc.

Although the ERK-CREB pathway is not the only mechanism by which deprivation circumstances can trigger neuroapoptosis, it nevertheless should be recognized that deactivation of the NMDA receptor-linked ERK-CREB pathway can result in a disruption of the homeostatic balance between pro- and anti-apoptosis proteins, thereby allowing pro-apoptosis proteins (culminating in Bax activation and translocation) to gain ascendancy, and trigger neuroapoptosis.

The present invention discloses a medicament composition for interceding in a manner that can stabilize the ERK-CREB pathway, thereby restoring the homeostatic balance between pro- and anti-apoptosis proteins in favor of cell survival. . It is important to note that these targets are part of the upstream stage of the apoptosis cascade, which offers important advantages as mentioned above.

For example, if suppression of ERK activation (which involves phosphorylation of the ERK protein) is counteracted by a pharmaceutical intervention that promotes ERK phosphorylation, this would abort the process of pathological neuroapoptotic signaling, very nearly at its inception. Suppression of NMDA receptor activity by an anesthetic drug could be carried out, allowing a desired analgesic and anesthetic outcome for a purposes such as surgery, but just as the ERK system is being negatively impacted, a counteracting positive force can stabilize the system, in ways that will prevent the pathological signaling cascade from propagating through any more steps.

The lithium ion offers one example of a candidate agent for achieving this purpose. It is known that lithium influences a number of intracellular signaling processes in ways that can affect various types of neurological processes (its most well-known use is for treating bipolar disorder, often called manic/depressive illness). For lithium to have its beneficial effects, it requires hours or preferably days of administration, during which time its influence in stabilizing intracellular signaling pathways will build up to stable levels. One such stabilizing influence that is documented in the literature is that lithium stimulates increased activation of the ERK MAP-kinase system (Einat et al., 2003), in ways that can be used, via CREB activation as mentioned above, to increased synthesis of the anti-apoptosis (pro-survival) protein, Bcl-2. Therefore, lithium could be administered a few days prior to surgery on a pediatric patient who must undergo prolonged anesthesia (such as to correct a cardiac anomaly). The lithium building during the presurgical period would stabilize the ERK-CREB pathway at a level that is somewhat higher than normal. This would help prevent a short-term decrease in that pathway from initiating, within a few hours, a signaling cascade that would lead to pathological neuroapoptosis.

The phospho-inositide 3 kinase (PI3K) system is a tyrosine kinase enzyme system that phosphorylates (and thereby activates) various protein molecules. The PI3K system is coupled to the NMDA receptor in such a manner that whenever the NMDA receptor is activated, this phosphorylating enzyme system is also activated, which in turn activates a system referred to as the AKT cascade. The AKT cascade sometimes feeds into the ERK cascade, but it also can act independently through several other pathways to influence cell survival, in some cases by direct interaction with the Bcl multidomain family of pro- and anti-apoptosis proteins. Accordingly, stimulation of the PI3K/AKT pathway promotes cell survival, whereas suppression of this pathway promotes apoptosis.

In unpublished studies, Olney and colleagues have found that within 30 minutes after administration of ethanol to infant mice there is a marked suppression of the activated isoform of AKT. These results are illustrated in the graph of FIG. 3C. Therefore, the PI3K/AKT pathway provides another target for therapeutic intervention, with the goal being to counteract the suppressive actions of anesthetic or similar drugs.

The protein kinase A (PKA) and protein kinase C (PKC) intracellular signaling pathways are also involved in regulating neuronal survival. Like the ERK system, they achieve this by influencing the balance between pro- and anti-apoptotic proteins in the Bcl multidomain family. As this is being written, it is not clear whether the PKA or PKC signaling pathways are altered by apoptogenic drugs used in pediatric and obstetric medicine; however, these pathways offer useful and promising targets for therapeutic intervention to prevent such drugs from triggering neuroapoptosis.

### REFERENCES

Beals, J.K., Carter, LB., Daniel-Johnson, J.A., Reiter, R.J., Jevtovic-Todorovic, V. Melatonin reduces apoptosis resulting from combined nitrous oxide, midazolam and isoflurane anesthesia in 7-day of rat pups. Soc. Neurosci. Abstract Neo. 635.6, 2003*.*
Dikianian K, Ishimaru MJ, Tenkova T, Labruyere J, Qin YQ, Ikonomidou C, and Olney JW Apoptosis in the in vivo mammalian forebrain. Neurobiol. Dis. 8: 359-379, 2001.
Dobbing J, Sands J The brain growth spurt in various mammalian species. Early Hum Dev, 3:79-84, 1979.
Einat H, Manji HK, Gould TD Du J, Chen G. Possible involvement of the ERK signalling cascade in bipolar disorder: behavioral leads from the study of mutant mice. Drug News Perspect. 16:453-463, 2003.
Naohiko Kobayashi; Shin-ichiro Mita; Kohtaro Yoshida; Takeaki Honda; Tsutomu Kobayashi; Kazuyoshi Hara; Shigefumi Nakano; Yusuke Tsubokou; Hiroaki Matsuoka Celiprolol Activates eNOS through the PDK-Akt Pathway and Inhibits VCAM-1 Via NF-B Induced by Oxidative Stress. Hypertension, 42:1004-1009, 2003.
Franks, N.P., Dickinson R., de Sousa, S.L., Hall, A.C., Lieb, W.R. How does xenon produce anaesthesia? Nature. 396(6709):324, 1998.
Harms C, Lautenschlager M, Bergk A, Freyer D, Weih M, Dirnagl U, Weber JR, Hörtnagl H. Melatonin is protective in necrotic but not in caspase-dependent, free radical-independent apoptotic neuronal cell death in primary neuronal cultures. FASEB J, 14(12):1814-24, 2000*.*
Ikonomidou, C, Bittigau, P., Ishimaru, M. J., Wozniak, D. F., Koch, C, Genz, K., Price, M.T., Stefovska, V., Hörster, F., Tenkova, T., Dikranian, K., and Olney, J. W. Ethanol-induced apoptotic neurodegeneration and fetal alcohol Syndrome. Science 287, 1056-1060, 2000.
Jevtovic-Todorovic V, Hartman RE, Izumi Y, Benshoff ND, Dikranian K, Zorumski CF, Olney JW and Wozniak DF. Early exposure to common anesthetic agents causes widespread neurodegeneration in the developing rat brain and persistent learning deficits. J. Neurosci, 23: 876-882, 2003.
Kerr, J.F.R., A.H. Wyllie, and A.R. Currie Apoptosis: a basic biological phenomenon with wideranging implications in tissue kinetics. British J. Cancer 26: 239-257, 1972.
Ma D, Hossain M, Pettet GK, Luo Y, Lim T, Akimov S, Sanders RD, Franks NP, Maze M. Xenon preconditioning reduces brain damage from neonatal asphyxia in rats. J Cereb Blood Flow Metab. July 20, 2005 (Epub ahead of print).
Nonaka, S., Katsube, N., Chuang, D.M. Lithium protects rat cerebellar granule cells against apoptosis induced by anticonvulsants, phenytoin and carbamazepine. J Pharmacol Exp Ther, 286(1):539-47, 1998*.*
Olney, J. W., Tenkova, T., Dikranian, K., Labniyere, J., Qin, Q. Y., & Dconomidou, C. Ethanol-induced apoptotic neurodegeneration in the developing C57BL/6 mouse brain.Dev. Brain Res. 133,115-126, 2002a.
Olney, J. W., Tenkova, T., Dikranian, K., Muglia, L. J., Jermakowicz, W. J., D'Sa, C, and Roth, K. A. Ethanol-induced caspase-3 activation in the in vivo developing mouse brain. Neurobiol. Dis. 9,205-219, 2002b.
Olney JW, Young C, Wozniak DF, Jevtovic-Todorovic V and Ikonomidou C. Do pediatric drugs cause developing neurons to commit suicide? Trends in Pharmacological Sciences (TiPS). 25(3):135-139, 2004.
Olney JW, Young C, Wozniak DF, Ikonomidou C., Jevtovic-Todorovic V Anesthesia-induced developmental neuroapoptosis: Does it happen in humans? Anesthesiology, 101, 273-275, 2004.
Young C, Klocke J, Tenkova T, Choi J, Labruyere J, Qin Y, Holtzman DM, Roth KA, Olney JW. Ethanol-Induced Neuronal Apoptosis in the in vivo Developing Mouse Brain is BAX Dependent: Cell Death and Differentiation. 10: 1148-1155, 2003.
Young C, Roth KA, Klocke BJ, West T, Holtzman DM, Labruyere J, Qin Y-Q, Dikranian K, Olney JW. Role of caspase-3 in ethanol-induced developmental neurodegeneration. Neurobiol Dis. 2005 (in press).
Zhu WZ, Zheng M, Koch WJ, Lefkowitz RJ, Kobilka BK, Xiao RP. Dual modulation of cell survival and cell death by beta(2)-adrenergic signaling in adult mouse cardiac myocytes. Proc Natl Acad Sci U S A. 98:1607-12, 2001.

## Claims

1. A medicament composition for use in a method of preventing pathological neuroapoptosis in fetuses, neonates or infants by exposure to an anesthetic drug, comprising lithium.

## Patentansprüche

1. Eine Medikamentenzusammensetzung zur Verwendung in einer Methode zur Verhinderung von pathologischer Neuroapoptose in Föten, Neugeborenen und Kleinkindern durch Exposition gegenüber einem Anästhetikum, die Lithium umfasst.

## Revendications

1. Une composition médicamenteuse comprenant du lithium pour utilisation dans une méthode de prévention de la neuro-apoptose pathologique chez le foetus, le nouveau-né ou le nourrisson par exposition à un anesthésiant.
